# FASCICULE DE BREVET EUROPEEN

(11) **EP 2 345 377 B1**
(45) Date de publication et mention de la délivrance du brevet: **08.05.2013**
(21) Numéro de dépôt: 11160989.7
(22) Date de dépôt: 23.02.2004
(51) Int. Cl.: A61B 17/122, A61B 17/128, A61B 17/08, A61B 17/12

(54) **Dispositif pour ligaturer une structure anatomique**
Vorrichtung zum abbinden anatomischer Strukturen
Device for ligating an anatomical structure

(30) Priorité: 24.02.2003 FR 0302216
(43) Date de publication de la demande: 20.07.2011
(62) Demande divisionnaire de: 04713568.6
(73) Titulaire: Sofradim Production, 01600 Trevoux (FR)
(72) Inventeur: Sgro, Jean-Claude, 21000, DIJON (FR)
(74) Mandataire: Cardy, Sophie Marie

(56) Documents cités:
- EP-A- 0 637 432
- EP-A1- 0 611 561
- DE-C1- 4 133 800
- US-A- 4 592 355
- US-A- 5 613 973
- US-A- 5 749 879
- US-A- 5 782 839
- US-A- 6 042 563

## Description

L'invention concerne un dispositif pour ligaturer une structure anatomique.

Le dispositif selon l'invention s'inscrit dans le cadre des matériels chirurgicaux permettant de réaliser des actes chirurgicaux notamment dans les espaces réduits en particulier sous coelioscopie. Cette technique mini-invasive comportant l'utilisation d'instruments fins, cylindriques, passés à travers la paroi abdominale sans utiliser de grande ouverture rend impossible l'utilisation des doigts ou de la main.

En ce qui concerne, par exemple, la résection chirurgicale de l'appendice iléo-cæcal, l'intervention est relativement simple lorsqu'elle est réalisée par voie ouverte conventionnelle ; il faut contrôler puis couper les vaisseaux qui alimentent l'appendice et qui sont inclus dans un feuillet anatomique nommé méso-appendice. Puis il faut lier la jonction de la base de l'appendice et du cæcum, partie inférieure du colon droit. Enfin, il faut couper l'appendice juste au-dessus de la ligature. La technique coelioscopique reprend ces temps opératoires, mais représente une technique plus difficile. La difficulté posée par la section du méso-appendice est résolue actuellement pour cette voie, soit par l'utilisation du bistouri électrique, soit par des clips métalliques. Mais l'utilisation d'énergie électrique, dangereuse, est à éviter. Quant à l'utilisation de clips métalliques, cela demande un matériel complexe, coûteux, parfois de taille inadaptée. La ligature de la base appendiculaire, a priori évidente, est souvent beaucoup plus difficile que prévue. Des noeuds coulants pré-montés sont disponibles mais ils sont souples, leur passage autour de l'appendice demande beaucoup d'attention, leur serrage doit être fait avec précaution, pour ne pas couper complètement l'appendice, mais il doit suffisamment être fort pour que le noeud ne se défasse pas et que les tissus ne glissent pas.

L'état de la technique est illustré par WO 01/34038, US 5,476,206 et US 5,433,721. Le préambule de la revendication 1 est basé sur le contenu du document DE 41 33 800 C1.

WO 01/34038 décrit un appareil permettant de saisir le moignon appendiculaire et de faire glisser sur lui un lien élastique. On procède de même pour le méso-appendice. Cela suppose que l'appendice soit assez mince et souple pour être inclus dans l'appareil, de même pour le méso-appendice, ou que la base de l'appendice et le méso aient déjà été coupés.

Dans certains cas particuliers où l'appendice est extrêmement remanié ou a augmenté de volume, il peut être nécessaire d'utiliser des appareillages plus complexes et coûteux, tels que ceux décrits dans les brevets US 5,476,206 et US 5,433,721. Ces appareillages permettent de poser deux rangées d'agrafes et de couper entre les deux rangées. Le résultat est certes satisfaisant mais on ne peut pas utiliser systématiquement ces appareillages car ils nécessitent des accès plus gros que ceux habituellement réalisés pour la résection chirurgicale de l'appendice et ils coûtent chers. Les accès nécessaires avec ces appareils ont en effet un diamètre compris entre 10 et 12 mm au lieu de 5 mm.

Des dispositifs individuels à usage unique peuvent convenir, car deux attaches peuvent suffire pour le traitement de lésions simples, d'une appendicectomie, ou de ligature isolée de vaisseaux sanguins.

L'invention concerne donc un dispositif pour ligaturer une structure anatomique oblongue, telle qu'un appendice iléo-cæcal, un méso-appendice ou un vaisseau sanguin, comportant un clip et un applicateur, formant un tout qui ne se dissocie qu'à la fin de la ligature, ce dispositif devant être manipulé d'une seule main pour crocheter la structure anatomique, la lier et éventuellement la couper.

Le document US 5 613 973 prévoit un dispositif pour ligaturer une structure anatomique oblongue comprenant, d'une part, un élément de ligature se présentant sous la forme d'une bande souple ayant une partie proximale et une partie distale, la partie proximale comportant un passant, et la partie distale présentant à son extrémité libre un crochet et étant susceptible de coulisser dans ledit passant après son introduction par l'extrémité distale du passant suite à une traction sur le crochet, et d'autre part un applicateur pour mettre en place cet élément de ligature et qui comporte :
- un tube cylindrique ayant une extrémité distale et une extrémité proximale.

Il n'existe pas jusqu'à présent d'autres matériels permettant de réaliser les différents temps chirurgicaux.

Le but de l'invention est de pallier à ce manque.

Selon l'invention, ce dispositif est caractérisé en ce que l'applicateur comporte en outre une tige métallique rectiligne qui s'étend dans ledit tube et qui comporte à son extrémité distale une lame ressort qui, en l'absence de contrainte, forme pratiquement un cercle dans le prolongement de ladite tige et dont le diamètre est compris entre cinq et douze fois le diamètre interne dudit tube, ladite lame étant susceptible de loger dans ledit tube à l'état déployé et de reprendre sa forme circulaire lorsqu'on la ressort par l'extrémité distale dudit tube,
- des moyens pour retenir, au moins durant son transport, ledit élément de ligature sur la face interne de ladite lame ressort, le passant étant disposé à la partie distale de ladite tige, et
- un passe-fil transitant par ledit passant et formant à l'avant de l'extrémité distale dudit passant une ganse dans laquelle s'introduit l'extrémité distale de l'élément de ligature lorsque la lame ressort forme un cercle, afin qu'une traction sur ledit passe-fil entraîne le crochetage de ladite ganse par le crochet, l'introduction de la partie distale de l'élément dans ledit passant et le serrage dudit élément de ligature.

Avantageusement les moyens pour retenir l'élément de ligature comportent une gaine en matériau souple présentant un conduit radialement extérieur dans lequel loge au moins la lame ressort et une rainure radialement intérieure dans laquelle loge au moins en partie l'élément de ligature, ladite rainure étant délimitée par deux lèvres retenant les bords longitudinaux dudit élément de ligature et étant susceptibles de s'écarter pour libérer ledit élément de ligature lors de son serrage.

Selon une variante avantageuse de l'invention, le passant est constitué par deux orifices ménagés dans la partie proximale de l'élément de ligature et par lesquels passe le passe-fil.

De préférence, l'extrémité distale du tube comporte en outre des moyens de découpe pour découper la portion distale de l'élément de ligature après serrage de ce dernier.

Ces moyens de découpe sont formés par les bords tranchants d'une encoche prévue à l'extrémité distale du tube.

D'autres avantages et caractéristiques ressortiront à la lecture de la description suivante faite à titre d'exemple et en référence aux dessins annexés dans lesquels :
la figure 1 montre en perspective un élément de ligature qui ne fait pas partie de l'invention ;
la figure 2 montre une deuxième variante de cet élément de ligature ;
la figure 3 montre une forme différente des éléments de clippage mâles et femelles ;
la figure 4 est une vue de dessus de l'élément de ligature qui ne fait pas partie de l'invention dans une position non verrouillée ;
la figure 5 est une vue de dessus de l'élément de ligature qui ne fait pas partie de l'invention de la figure 4 en position verrouillée ;
les figures 6 et 7 sont des vues de la face interne d'une branche de l'élément de ligature qui ne fait pas partie de l'invention, qui montrent des variantes de réalisation des échancrures ;
la figure 8 montre une troisième variante de l'élément de ligature qui ne fait pas partie de l'invention ;
la figure 9 est une vue de dessus de l'élément de ligature de la figure 8 en position verrouillée ;
la figure 10 montre une quatrième variante simplifiée de l'élément de ligature qui ne fait pas partie de l'invention ;
la figure 11 montre un applicateur qui ne fait pas partie de l'invention et qui est particulièrement adapté pour mettre en place l'élément de ligature montré sur les figures 1 et 2 ;
la figure 12 montre en perspective l'extrémité distale de l'applicateur de la figure 11 sans le coulisseau ;
la figure 13 est une coupe selon un plan médian transversal de l'extrémité distale de l'applicateur sans le coulisseau ;
la figure 14 est une coupe selon un plan perpendiculaire à l'axe de l'applicateur de l'extrémité distale de ce dernier avec le coulisseau ;
la figure 15 est une vue en perspective du coulisseau ;
la figure 16 montre une variante de l'applicateur qui ne fait pas partie de l'invention ;
la figure 17 montre une variante de réalisation de la rainure de la paroi latérale du logement de l'élément de ligature ;
la figure 18 montre en perspective un autre élément de ligature qui ne fait pas partie de l'invention ;
la figure 19 montre une variante de cet autre élément de ligature qui ne fait pas partie de l'invention ;
la figure 20 montre une manière possible de ligaturer une structure anatomique par l'élément de ligature souple montré sur la figure 19, sans système d'application, en utilisant une instrumentation coelioscopique standard;
la figure 21 montre l'extrémité distale d'un applicateur spécifique pour mettre en place l'élément de ligature montré sur la figure 18 ;
la figure 22 montre en perspective l'extrémité distale d'un applicateur spécifique pour mettre en place l'élément de ligature semblable à celui montré sur la figure 19 ;
la figure 23 est une vue de dessus d'un mode de réalisation de l'élément de ligature selon l'invention ;
la figure 24 est une vue latérale de l'élément de ligature de la figure 23 ;
la figure 25 montre l'applicateur pour mettre en place l'élément de ligature selon le mode de réalisation de l'invention, avant l'intervention chirurgicale ;
la figure 26 est une vue en coupe de l'applicateur selon la ligne XXVI-XXVI de la figure 25 ;
la figure 27 est une vue latérale de l'applicateur de la figure 25 au cours de l'intervention chirurgicale ;
la figure 28 est une coupe du support de l'élément de ligature selon la ligne XXVIII-XXVIII de la figure 27 ;
la figure 29 est une coupe transversale du tube et du passant prise selon la ligne XXIX-XXIX de la figure 27 ;
la figure 30 est une vue latérale de l'applicateur après serrage de l'élément de ligature ;
la figure 31 montre les moyens de découpe de la partie distale de l'élément de ligature après serrage ; et
la figure 32 montre une variante de réalisation du mode de réalisation de l'élément de ligature selon l'invention.

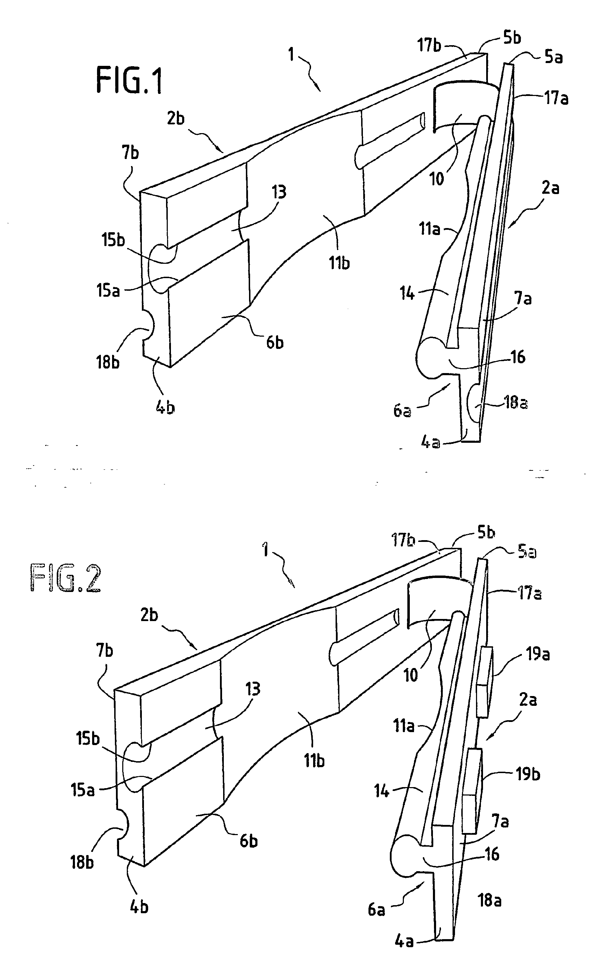

Les figures 1 à 10 qui ne font pas partie partie de l'invention, montrent un élément 1 pour ligaturer notamment d'une structure anatomique oblongue, qui comportent deux branches rigides 2a, 2b présentant chacune une extrémité proximale 4a, 4b, une extrémité distale 5a, 5b, une face interne 6a, 6b et une face externe 7a, 7b, l'indice a des références 4 à 7 se rapportant à la première branche 2a, et l'indice b des références 4 à 7 se rapportant à la deuxième branche. Une bande mince flexible 10 relie les extrémités distales 5a, 5b des deux branches 2a et 2b. L'élément de ligature 1 est réalisé de préférence par moulage ou par découpe d'un matériau approprié répondant aux exigences médicales, et notamment d'un matériau résorbable, et la bande mince flexible 10 est calculée de telle manière qu'au repos, les deux branches 2a et 2b forment un angle aigu.

Ainsi que cela se voit clairement sur les figures 1 et 2, qui concernent des éléments de ligature 1 destinés à être mis en place par un applicateur décrit plus loin dans le présent mémoire, les faces internes 6a et 6b des branches 2a et 2b présentent chacune une échancrure 11a, 11b transversale qui formera un canal transversal 12 destiné à enserrer une portion de la structure anatomique.

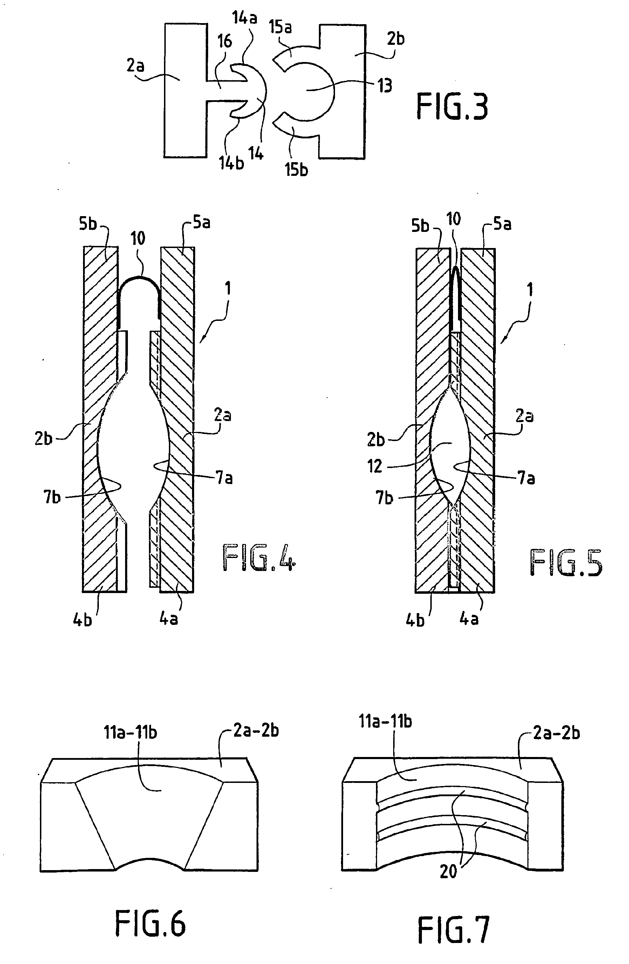

Dans la face interne 6b de la deuxième branche 2b, est ménagée une gorge longitudinale 13 destinée à recevoir de force une paroi cylindrique 14 prévue sur la face interne 6a de la première branche 2a. La gorge 13 est réalisée à contre-dépouille et elle présente sur la face interne 6b des lèvres 15a, 15b qui se déforment élastiquement lors de l'introduction de la paroi cylindrique 14, cette introduction étant obtenue en rapprochant les deux branches 2a, 2b et en exerçant sur elles des efforts de compression. La paroi cylindrique 14 est raccordée à la première branche 2a par une paroi 16 qui a une épaisseur au plus égale à la distance qui sépare les deux lèvres 15a et 15b. Le diamètre de la paroi cylindrique 14 est au plus égal au diamètre de la gorge 13. Les dimensions de la gorge 13 et de la paroi cylindrique 14 sont calculées de telle manière que la paroi cylindrique 14 puisse être introduite dans la gorge 13 par clippage, les faces internes 6a et 6b des deux branches 2a et 2b étant alors accolées, ainsi que cela est visible sur la figure 5, où l'on voit clairement le canal transversal 12 formé par les échancrures 7a et 7b.

Sur les figures 1 et 2, on voit que les branches 2a et 2b présentent à leurs extrémités distales 5a et 5b des portions d'extrémité 17a et 17b situées au-delà du raccordement de la bande mince flexible 10. Ces deux portions d'extrémité 17a et 17b sont destinées à permettre l'écartement des deux branches 2a et 2b lors de la mise en place par l'applicateur décrit plus loin afin de pouvoir crocheter la structure anatomique.

Ainsi que cela est montré sur la figure 1, les deux branches 2a et 2b présentent sur leurs faces externes 7a et 7b, des rainures longitudinales 18a, 18b, servant au guidage d'un coulisseau de l'applicateur. La rainure longitudinale 18b de la branche 2b au moins présente des protubérances non montrées sur les figures 1 et 2, qui par coopération avec un coulisseau de l'applicateur permettront de rapprocher les deux branches 2a et 2b et l'introduction de la paroi cylindrique 14 dans la gorge 13. Ces protubérances sont formées notamment près de l'extrémité proximale 4a, et éventuellement à d'autres endroits. La portion d'extrémité 17b de la branche 2b présente également dans la rainure 18b une protubérance pour écarter la branche 2b de la branche 2a.

La figure 2 montre sur la face externe 7a de la branche 2a des protubérances 19a servant au maintien de l'élément de ligature 1 sur l'applicateur, ces protubérances coopérant également avec le coulisseau pour comprimer les deux branches 2a et 2b lors de l'opération de clipsage, ainsi que cela est décrit plus en détail dans la suite du présent mémoire.

La figure 3 montre une variante de réalisation des moyens de clipsage. La gorge 13 comporte des parois élastiques se terminant par les lèvres 15a et 15b. La paroi cylindrique 14 comporte également des lèvres 14a et 14b flexibles qui se rapprochent lors de leur passage entre les lèvres 15a et 15b.

La figure 4 montre l'élément de ligature dans une position de transport vers le lieu de son application. Les deux branches 2a et 2b sont parallèles entre elles, mais non liées par les éléments de clippage 13 et 14.

Dans cette position, l'élément de ligature 1 présente une section relativement faible.

La figure 5 montre l'élément de ligature 1 après fixation des deux branches 2a et 2b entre elles. La section du canal transversal 12 est adaptée à la section de la structure anatomique sur laquelle l'élément de ligature 1 est implanté.

Les figures 6 et 7 montrent deux variantes de réalisation des échancrures 11a et 11b. La figure 6 montre une échancrure ayant une forme évasée vers le haut, tandis que la figure 6 montre une échancrure ayant des génératrices parallèles mais comportant des demi-joncs 20 dans des plans transversaux qui permettent de bien enserrer la structure anatomique.

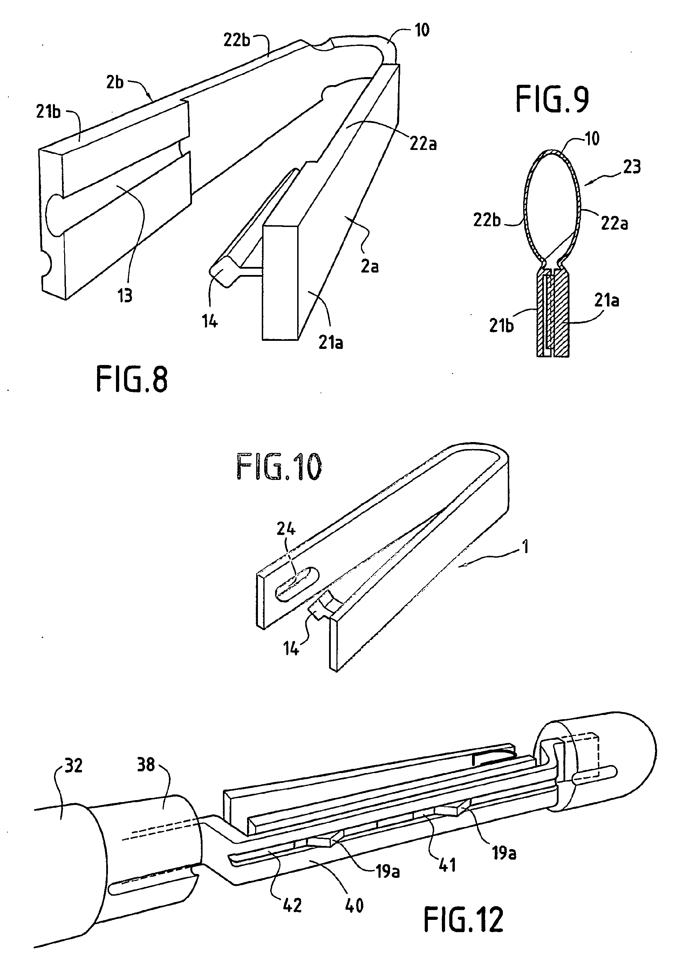

Les figures 8 et 9 montrent un élément de ligature 1 dans lequel seules les portions proximales 21a et 21b des branches 2a et 2b comportent des moyens de clippage 13 et 14, tels que ceux décrits ci-dessus, les autres portions 22a, 22b des branches 2a et 2b étant plus minces et susceptibles de se déformer élastiquement pour former, avec la charnière 10, une boucle 23, après clippage ainsi que cela est représenté sur la figure 9.

La figure 10 montre une variante simplifiée de l'élément de ligature 1, dans lequel la gorge 13 est remplacée par une fenêtre 24 à travers laquelle vient se bloquer l'élément de clippage mâle 14.

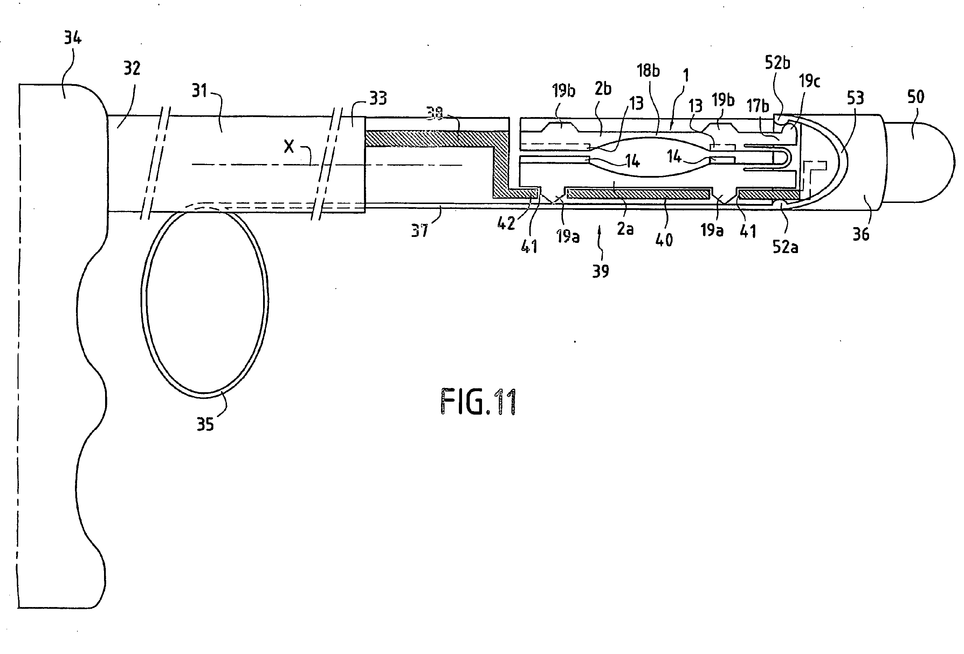

Les figures 11 à 17 montrent en détail un applicateur 30 pour mettre en place l'élément de ligature 1, montré sur les figures 1 et 2, et ne font pas partie de l'invention.

L'applicateur 30 comporte un tube cylindrique 31 qui présente une extrémité proximale 32 et une extrémité distale 33. Le corps 31 a un petit diamètre, par exemple 5 mm, et est destiné à passer par une canule disposée, au moyen d'un trocart à travers la paroi d'un patient.

L'extrémité proximale 32 du corps 31 est de préférence équipée d'une poignée de préhension 34. En regard de la poignée de préhension, le corps 31 est équipé d'un organe de manoeuvre 35 monté axialement mobile sur le corps 31 et relié à un coulisseau 36, décrit plus en détail plus loin dans le présent mémoire, par une tige 37 montée coulissante dans le corps 31.

Le corps 31 présente à son extrémité distale 33, une portion 38, de diamètre rétréci, susceptible de recevoir le coulisseau 36. Cette portion rétrécie 38 est prolongée par un logement 39 destiné à accueillir un élément de ligature 1. Le logement 39 est constitué essentiellement par une plaque en forme de U qui comporte une paroi latérale ou de fond 40 parallèle à l'axe X du corps 31 et contre laquelle la face externe de la première branche 2a de l'élément de ligature 1 est retenue au moyen des protubérances 19a logeant dans des fenêtres 41 ménagées dans la paroi latérale 40, ces fenêtres débouchant elles-mêmes dans une rainure longitudinale 42 ménagée sur la face externe de la paroi latérale 40. Les protubérances 19a et 19b émergent dans la rainure 42.

Ainsi que cela est montré sur la figure 11, la face externe de la deuxième branche 2a présente également dans la rainure 18b, deux protubérances 19 opposées aux deux protubérances 19a, chaque paire de protubérance 19a, 19b étant positionnée axialement ou longitudinalement au niveau d'une paire de moyens de clippage 13 et 14.

On voit également sur cette figure que la rainure 18b présente à l'extrémité libre du prolongement 17b une troisième protubérance 19c.

La référence 50 désigne un embout mousse disposé à l'extrémité avant du logement 39.

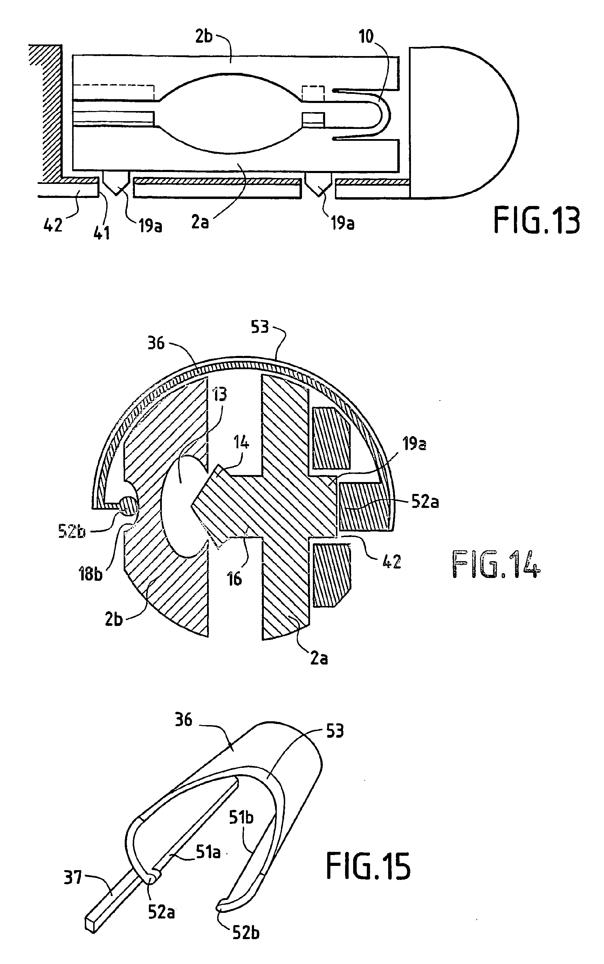

Le coulisseau 36 montré en détail sur la figure 15 se présente sous la forme d'une gouttière semi-cylindrique qui présente deux lèvres longitudinales 51a, 51b. L'une des extrémités de ces lèvres présente un bossage 52a, 52b, les deux bossages 52a et 52b étant en vis-à-vis et coulissant respectivement dans la rainure 18b de l'élément de ligature 1 et dans la rainure 42 de la paroi latérale 40 du logement 39.

La portion rétrécie 38 et l'embout de mousse 50 comportent également des rainures dans le prolongement des rainures 18b et 42.

Comme on le voit en outre sur les figures 11 et 15, le bord 53 du coulisseau qui s'étend entre les deux bossages 52a et 52b est disposé dans un plan oblique et est biseauté afin de former un outil de découpe de la structure anatomique après la ligature de cette dernière.

L'applicateur 30 décrit ci-dessus est utilisé de la manière qui va être décrite ci-après.

L'élément de ligature 1 est disposé dans le logement 39, les protubérances 19a et 19b étant positionnées dans les fenêtres 41 et le coulisseau 36 étant au niveau de l'embout mousse 50.

En déplaçant légèrement le coulisseau vers l'extrémité distale 32 du corps 31, le bossage 52b passe au-dessus de la protubérance 19c et vient se positionner entre la protubérance médiane 19b et la protubérance 19c. L'élément de ligature 1 est alors emprisonné dans le logement 39, dans la position de transport, et l'applicateur 30 est prêt à l'emploi.

Lorsqu'on a introduit l'applicateur 30 par une canule dans le corps du patient, on doit ouvrir l'élément de ligature 1 afin de crocheter la structure anatomique. Pour ce faire, l'opérateur déplace manuellement l'organe de manoeuvre 35 vers l'extrémité distale 33. Le coulisseau se déplace axialement, et le bossage 52b appuie sur la protubérance 19c du prolongement 17b de la deuxième branche 2b, ce qui écarte la deuxième branche 2b de la première branche 2a toujours maintenue contre la paroi latérale 40.

Dès qu'on a crocheté la structure anatomique, l'opérateur tire manuellement sur l'organe de manoeuvre 35, le coulisseau 36 se déplace axialement dans la direction de l'extrémité proximale 32, et les bossages 52a et 52b rencontrent les premières paires de protubérances 19a, 19b, ce qui rapproche les portions distales des branches 2a, 2b et entraîne le clippage d'une paroi cylindrique 14 dans la gorge adjacente.

En poursuivant la traction de l'organe de manoeuvre 35, les bossages 52a et 52b viennent au contact des protubérances 19a et 19b situées au voisinage des extrémités proximales 4a, 4b des deux branches 2a et 2b, et assurent le clippage des portions proximales des branches 2a et 2b lors du franchissement de ces protubérances. L' élément de ligature 1 est alors en place. En poursuivant son déplacement vers l'extrémité proximale 32, le bord biseauté 53 du coulisseau 36 coupe la structure anatomique au-dessus de la ligature réalisée.

Lorsque le coulisseau 36 sera positionné sur la portion rétrécie 38 du corps cylindrique 31, l'élément de ligature 1 est libéré, et ce d'autant plus facilement que le bossage 52a aura dégagé en partie les protubérances 19a et 19b des fenêtres 41 lors du déplacement axial du coulisseau 36.

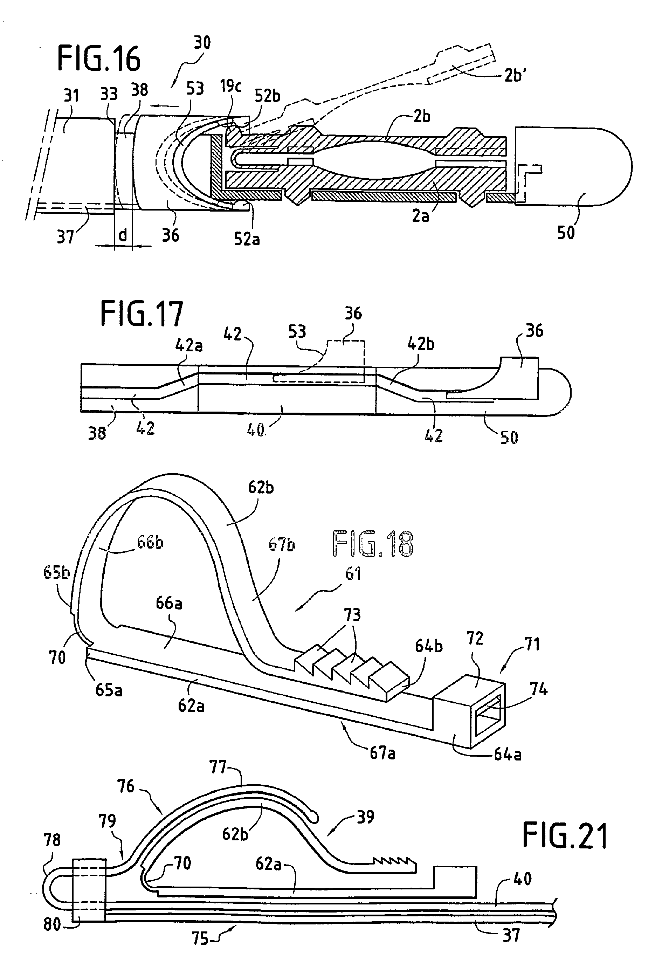

La figure 16 montre une variante de réalisation de l'applicateur 30 décrit ci-dessus.

Dans cette variante, les extrémités proximales 4a, 4b des deux branches 2a, 2b de l'élément de ligature 1 sont disposées au voisinage de l'embout mousse 50, et le coulisseau 36 présente les bossages 52a, 52b et le bord coupant 53 du côté de l'embout mousse 50.

Sur la figure 16, on a montré en trait plein la position de l'élément de ligature 1 et du coulisseau 36 lorsque l'applicateur 30 est prêt à être utilisé. Le bossage 52b se trouve à l'avant de la protubérance 19c. Le coulisseau 36 est au niveau de la portion rétrécie 38, sa face arrière étant à une distance d de l'extrémité distale du corps 31. Si l'opérateur recule le coulisseau 36 de la distance d, le bossage 52b vient appuyer sur la protubérance 19c. La branche 2b s'écarte de la branche 2a et prend la position montrée en pointillé et référencée 2b'. Au lieu de former un crochet, l'élément de ligature 1 se présente sous la forme d'une pince que l'opérateur peut positionner sur la structure anatomique. Par déplacement du coulisseau 36 vers l'embout mousse 50, on provoque successivement la fermeture et le clippage des deux branches 2a et 2b, puis la découpe de la structure anatomique enserrée dans le canal transversal 12 de l'élément de ligature 1.

Il est à noter que lorsque l'élément de ligature 1 est disposé dans le logement 39 en position de transport, c'est-à-dire les deux branches 2a et 2b étant parallèles entre elles, mais non verrouillées, la section transversale de l'ensemble constitué par le logement 39, l'élément de ligature 1 et le coulisseau 36 est au plus égale à celle du corps cylindrique 31 afin de passer par la canule du trocart.

Il est à noter, en outre, que la rainure 42 ménagée sur la face externe de la paroi latérale 40 du logement 39 sur la portion rétrécie 38 et sur l'embout mousse 50 peut présenter des tronçons 42a, 42b obliques, afin d'éloigner le bord tranchant 53 de l'élément de ligature 1 lors de la coupe de la structure anatomique, ainsi que cela est montré sur la figure 17.

La figure 18 montre un élément de ligature 61 qui ne fait pas partie de l'invention et qui comporte une première branche 62a semi-rigide et une deuxième branche 62b également semi-rigide présentant chacune une extrémité proximale 64a, 64b, une extrémité distale 65a, 65b, une face interne 66a, 66b, et une face externe 67a, 67b. Une bande mince flexible 70 relie les extrémités distales 65a, 65b des deux branches 62a et 62b. Cet élément de ligature 61 est également réalisé, de préférence, par moulage d'un matériau approprié, répondant aux exigences médicales, en une ou plusieurs pièces assemblables.

La première branche 62a présente sur sa face interne 66a au voisinage immédiat de son extrémité proximale 64a un passant 71, de section sensiblement rectangulaire, ayant une paroi 72 distante de la face interne 66a.

La deuxième branche 62b présente sur la face externe 67b de sa portion proximale une pluralité de crans 73 transversaux. La section du conduit 74 du passant 72 est calculée de telle manière que la portion proximale de la deuxième branche, introduite par l'extrémité distale du conduit 74, puisse coulisser de force ou à légère friction dans le conduit 74, l'un des crans 73 venant ensuite en appui sur l'extrémité proximale de la paroi 72 pour empêcher un retour arrière de la portion proximale, ce qui entraîne la fixation des portions proximales des deux branches 62a et 62b entre elles.

Les crans 73 peuvent être disposés latéralement sur la portion proximale de la deuxième branche 62b ou être remplacés par de simples aspérités, picots ou rugosités, qui entraînent des forces de frottement assurant le serrage.

La figure 21 montre l'extrémité distale 75 d'un applicateur 30 pour mettre en place l'élément de ligature 61 et qui ne fait pas partie de l'invention. Cet applicateur 30 est sensiblement semblable à celui décrit plus haut et servant à mettre en place l'élément de ligature 1. Les seules différences concernent le logement 39 de l'élément de ligature 61 et le coulisseau 36 relié à l'organe de manoeuvre 35 par une tige 37 mobile axialement.

Le logement 39 comporte une paroi latérale 40, et un ressort à lame 76 qui présente en regard de la paroi latérale 40 une surface 77 bombée reliée à l'extrémité distale 78 de la paroi latérale 40 par un arceau 79. Le coulisseau 36 est constitué d'une bague 80 fixée à l'extrémité distale de la tige 37. La bague 80 est disposée dans un plan perpendiculaire à l'axe X de l'applicateur 30, et elle est traversée par la paroi latérale 40 et le ressort à lame 76.

Lors du déplacement axial du coulisseau 76, le ressort à lame 76 et la paroi latérale 40 coulissent dans l'alésage de la bague 80.

La surface bombée 77 se raccorde à l'arceau 78 par une zone de raccordement 79 à courbure inversée.

L'élément de ligature 61 est disposé entre la paroi latérale 40 et le ressort à lame 76. La première branche 62a est en appui contre la paroi latérale 40 et la deuxième branche 62b est en appui contre la surface bombée 77. La bande flexible mince 70 est disposée en regard de l'arceau 78.

Lorsque la bague 80 est dans sa position la plus distale, la deuxième branche 62b est écartée au maximum de la première branche 62a et il est possible de crocheter une structure anatomique à ligaturer.

Lorsque l'opérateur tire sur l'organe de manoeuvre 35, la surface bombée 77 se rapproche de la paroi latérale 40 et la portion d'extrémité proximale de la deuxième branche 62b vient se mettre en regard de l'orifice 74 du passant 71. L'élément de ligature 1 est alors dans la position de transport et l'applicateur 30 peut être introduit dans la canule. La section du logement 39 est alors au plus égale à la section du corps cylindrique 31.

Pour ouvrir l'élément de ligature, l'opérateur repousse l'organe de manoeuvre 35, puis, après avoir crocheté la structure anatomique, il tire sur l'organe de manoeuvre, afin de rapprocher la deuxième branche 62b de la première branche 62a, comme représenté sur la figure 21. En tirant davantage sur l'organe de manoeuvre 35, la portion crantée de la deuxième branche 62b pénètre et coulisse dans l'orifice 74 du passant 71, ce qui enserre la structure anatomique dans le canal transversal de l'élément de ligature 61.

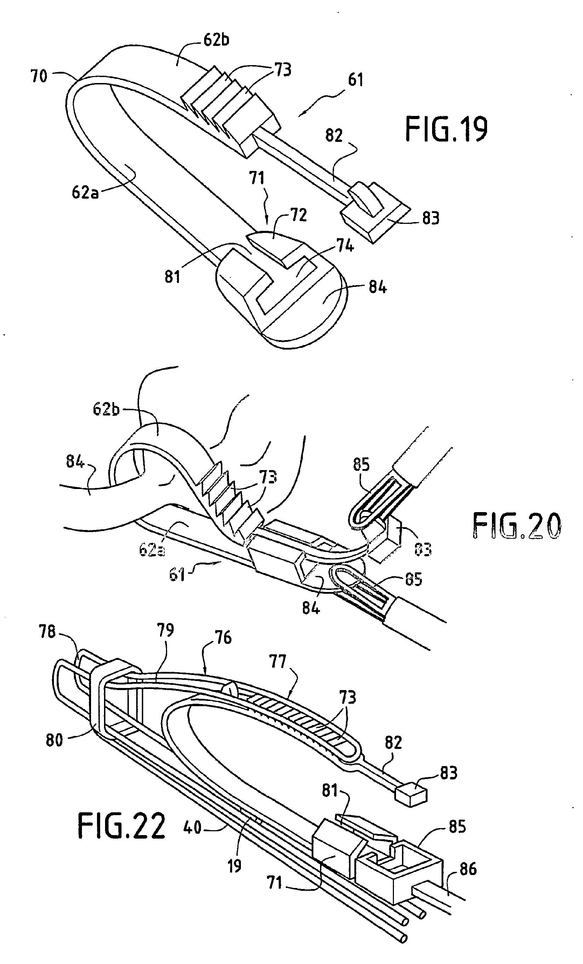

La figure 19 montre une variante de l'élément de ligature 61 et ne fait pas partie de l'invention. Cette variante diffère de celle montrée sur la figure 18, par une fente longitudinale 81 ménagée dans la paroi 72 du passant 71 et par une languette 82 située dans le prolongement de la portion crantée de la deuxième branche 62b. Cette languette 82 a une longueur légèrement supérieure à la longueur du passant 71 et peut être introduite dans le conduit 74 par coulissement à travers la fente longitudinale 81. La languette 82 se termine par un moyen de préhension 83. L'extrémité proximale de la première branche 62a peut également comporter un moyen de préhension 84. La deuxième branche 62b est dans ce cas souple. L'ensemble 61 est ,dans ce cas, constitué en totalité en matériau souple pour se comporter comme une ligature.

La figure 20 montre une manière de monter l'élément de ligature 61 décrit ci-dessus autour de la structure anatomique en prenant les languettes 84 et 85 au moyen de pinces coelioscopiques.

Cet élément de ligature 61 peut être également mis en place au moyen de l'applicateur 30 décrit plus haut. Lorsque par déplacement axial de la bague 80, la surface bombée 77 se rapproche de la paroi latérale 40, la languette 82 passe d'abord par la fente 81, puis la portion crantée coulisse dans le conduit 74 du passant 71.

La figure 22 montre une variante de réalisation de l'applicateur 30 qui ne fait pas partie de l'invention. Cet applicateur 30 présente un réceptacle 85 au voisinage du logement 39 dans lequel le moyen de préhension 83 situé à l'extrémité de la languette vient se loger lorsque la languette 82 est disposée dans le conduit 74 du passant 71. Ce réceptacle 85 est relié par une deuxième tige 86 à un deuxième organe de manoeuvre, non montré sur les dessins, situé à l'extrémité proximale du corps cylindrique 31 de l'applicateur 30.

Comme on le voit sur la figure 22, la paroi latérale 40 et le ressort à lame 76 peuvent être réalisés au moyen de fils à ressorts de section cylindrique ou aplatie. Ce ressort a un rôle important : il permet de manoeuvrer la branche 62b à laquelle il est solidarisé par le ou les ergots ; sa forme en 78 crée plusieurs plateaux successifs, la bague 80 étant poussé à l'extrémité de l'instrument , la partie 76 préformée ,se relève entraînant avec elle la branche 62b ; lorsque la bague 80 est tirée sur le cran 79 la partie 76 amène la branche 62b au contact du passant 71 et du réceptacle 85.Cette position est réputée être la position de transport ou d'introduction dans un trocart d'accès, en sachant que la boucle formée par les 2 branches 62 a et 62b sera aplatie au passage dans le trocart et que la partie effilée 82, d'une longueur calculée pour ce faire, coulissera librement dans la fente 81,et dans le réceptacle 85 dont la paroi proximale 85 bis sera suffisamment éloignée pour permettre les déplacements des parties 82 et 83. La partie rectiligne 40 du ressort contre laquelle coulisse l'axe 75 , reçoit les ergots 19 qui maintiennent dans le logement la branche 62a et la partie postérieure du passant 71.Ce dernier vient en appui sur un relief 87 du manche de l'applicateur de telle sorte que lors de la traction du moyen de préhension 83 par la partie échancrée du réceptacles 85 , le passant 71 puisse rester fixe tandis que la branche 62b et les crans 73 sont tirés à travers la partie interne du passant 71.Lorsque la boucle formée par les branches 62a et 62b est suffisamment refermée sur la structure anatomique à contrôler, l'axe 86 est repoussé vers la partie distale de l'instrument pour dégager l'élément 61 de son applicateur.

De préférence, l'élément de ligature 1 ou 61 est pré monté en atelier sur l'applicateur 30,en engageant les ergots 19 dans des espaces, prévus à cet effet, dans la paroi du ressort 40 et 76, pour constituer un ensemble stérile prêt à l'emploi.

Plusieurs parties de l'applicateur 30 et notamment le corps cylindrique 31 sont réalisés en un matériau plastique.

La mise en place de l'élément de ligature 1 au moyen de l'applicateur 30 se fait par l'opérateur sous le contrôle optique d'une vidéo, ce qui permet d'économiser une pince et un trocart supplémentaire, par rapport à la technique actuelle.

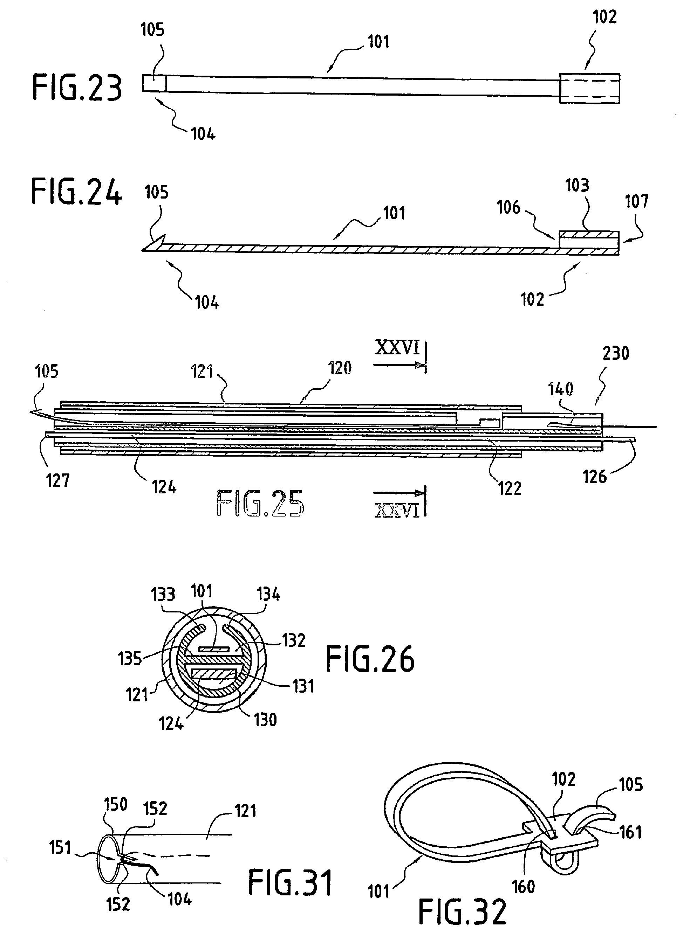

Les figures 23 et 24 montrent un élément de ligature 101 selon l'invention qui se présente sous la forme d'une bande souple, relativement longue, dont la partie proximale 102 présente sur sa face supérieure ou interne, un passant 103, et dont l'extrémité de la partie distale 104 comporte sur sa face supérieure ou interne un crochet 105. Le crochet 105 est destiné à être introduit, par traction, dans le passant 103 par l'extrémité distale 106 de ce dernier. En continuant à tirer sur le crochet 105, la partie distale 104 pénètre et coulisse dans le passant 103, et la partie de la bande voisine du passant 103 forme une boucle de serrage autour de l'élément anatomique à ligaturer, la partie distale 104 pouvant alors être coupée devant l'extrémité proximale 107 du passant 103. La face inférieure de l'élément de ligature 101 peut présenter dans sa zone médiane des crans qui permettent le blocage dans le passant 103 après serrage.

Les figures 25 à 30 montrent l'applicateur 120 utilisé dans le cadre de l'invention pour mettre en place l'élément de ligature 101 autour d'un élément anatomique 110.

Cet applicateur 120 comporte essentiellement un tube 121 rigide et rectiligne dont le diamètre extérieur est au plus égal à 5 mm et dont le diamètre intérieur est voisin de 4 mm.

Dans ce tube 121 peut coulisser une tige métallique 122 rectiligne, réalisée sous la forme d'une bande ayant une largeur de 1 à 3 mm et une épaisseur de 4/10 à 8/10 mm, qui présente à son extrémité distale 123 une lame ressort 124, formée à chaud, qui, en l'absence de contrainte, se présente sous la forme d'un cercle presque complet qui a un diamètre compris entre cinq et douze fois le diamètre intérieur du tube 121 et dont la section est de préférence identique à la section de la tige 122. Le diamètre de la lame ressort 124 est par exemple de 5 cm environ et sa circonférence est alors comprise entre 15 et 20 cm.

La tige 122 et la lame ressort 124 sont réalisées de préférence dans le même matériau et tirées de la même bande métallique par exemple en alliage de Nickel-Titane, cet alliage permettant de réaliser à chaud des ressorts ayant une forme circulaire, qui peuvent retrouver cette forme circulaire initiale après avoir été escamotés ou mis dans un état déployé quasi rectiligne dans le tube rigide et rectiligne 121.

Lorsque la lame ressort 124 forme un cercle, ce cercle et la tige 122 sont dans le même plan. La lame ressort 124 est alors dans le prolongement de la tige 122 à l'extérieur du tube 121, et la jonction entre la partie distale 123 de la tige 122 et la lame ressort 124 est calculée pour que le prolongement de la tige 122 coupe le cercle au voisinage de son centre 125 et se trouve éloigné de ce centre 125 d'une distance égale au plus à deux tiers du rayon du cercle. L'angle extérieur formé par la tige 122 et la tangente au cercle au point de jonction 123 est compris entre 90° et 180° par exemple.

La fonction de la lame ressort 124 est d'entourer la structure anatomique 110 par l'élément de ligature 101 décrit ci-dessus.

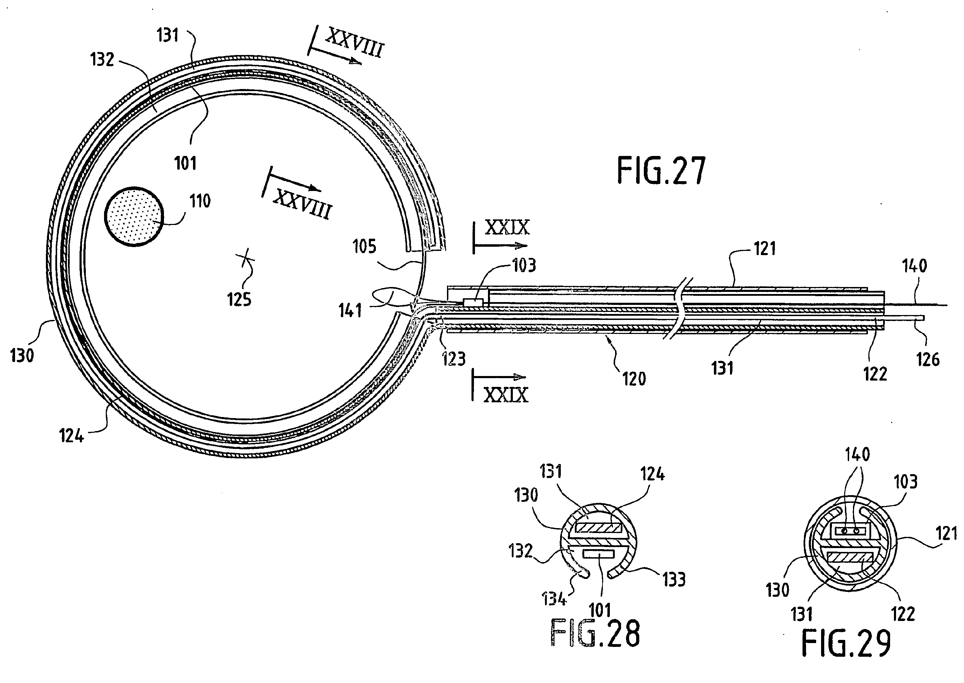

A cet effet, l'applicateur 120 comporte en outre une gaine 130 en matériau plastique souple ayant une surface lisse pour pouvoir coulisser facilement dans le tube 121, et qui comporte un conduit 131 radialement extérieur dans lequel loge la lame ressort 124 et la tige métallique 122, et, sur la face radialement interne de la lame ressort 124, une rainure 132 dans laquelle l'élément de ligature 101 loge notamment au cours de son transport autour de la structure anatomique 110 ainsi que cela est montré sur les figures 27 et 28.

En pratique, comme on le voit sur les figures 26 et 28, la gaine 130 se présente sous la forme d'un tube fendu sur une partie de sa longueur et ayant une cloison interne 135 délimitant le conduit 131 et la rainure 132.

La rainure 132 est délimitée par deux lèvres 133 et 134 et par une portion de paroi 135 du conduit 131.

La fonction des lèvres 133 et 134 est de retenir les bords longitudinaux de l'élément de ligature 101, mais ces lèvres peuvent s'écarter pour libérer l'élément de ligature 101 lors du serrage de ce dernier.

L'élément de ligature 101 est disposé dans la rainure 132 de telle manière que le passant 103 se trouve au-dessus de la partie distale de la tige métallique 122, sa partie distale 104 et notamment le crochet 105 se trouvant au-delà de l'extrémité libre de la lame ressort 124, c'est-à-dire en dessous du plan de la tige 122, lorsque la lame ressort 124 forme un cercle, ainsi que cela est montré sur la figure 27.

L'applicateur 120 est en outre équipé d'un passe-fil 140 ou fil double qui loge en partie dans le tube 121, traverse le passant 103 et forme une boucle ou ganse 141 au-delà de l'extrémité distale 106 du passant 103.

La gaine 130 et les éléments 122, 123 et 124 sont solidaires, on décrit cet ensemble par le numéro 230. Le passe-fil, bien que malléable est solidaire lors de la poussée de l'ensemble 230.

En exerçant une traction sur l'extrémité proximale de l'ensemble 230, la jonction entre la tige 122 et la lame ressort 124 se déforme élastiquement et pénètre dans le tube 121 par son extrémité distale 127. On conçoit que toute la lame ressort 124 peut être escamotée dans le tube 121 comme cela est montré sur la figure 25.

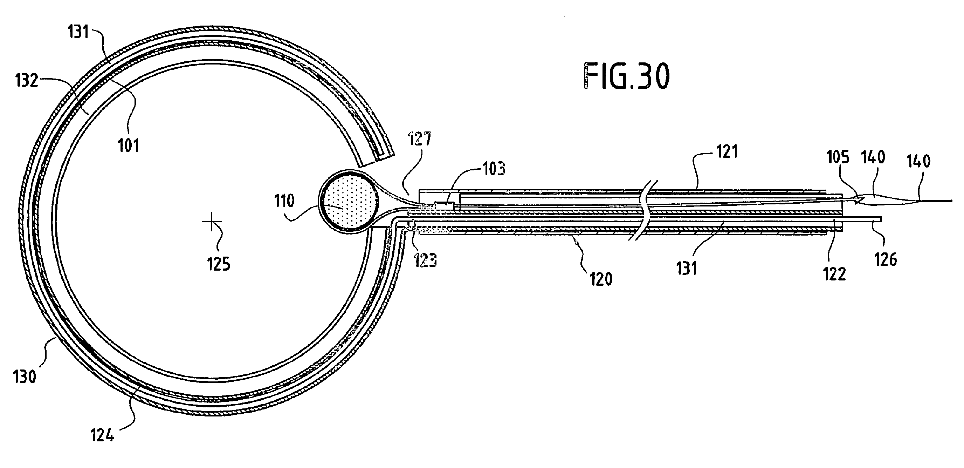

Ainsi que cela est montré sur la figure 27, le crochet 105 se trouve sous le plan de la boucle 141 du passe-fil 140. En exerçant une traction sur la partie externe du passe-fil 140 qui ressort par l'extrémité proximale du tube 121, la boucle 141 coopère avec le crochet 105 et permet de faire passer ce crochet 105 et la partie distale 104 de l'élément de ligature 101 dans le passant 103 via son extrémité distale 106. Lors de cette traction, le diamètre de la boucle formée par l'élément de ligature 101 diminue et l'élément de ligature 101 s'échappe de la rainure 132 par écartement des lèvres 133 et 134. Une traction complémentaire permet le serrage de l'élément de ligature 101 autour de la structure anatomique 110 ainsi que cela est montré sur la figure 30.

En exerçant une poussée sur la tige 122, le passant 103 sort du tube 121. Il est alors possible de couper la partie distale 104 de l'élément de ligature 101 près de l'extrémité proximale 107 du passant 103.

Ainsi que cela est montré sur la figure 31, l'extrémité distale 150 du tube 121 présente une encoche 151 comportant des bords 152 tranchants susceptibles de couper la partie distale 104 retenue par le passe-fil 140.

La figure 32 montre une variante de réalisation de l'élément de ligature 101 selon l'invention qui diffère de celui montré sur les figures 23 et 24 par le fait que le passant 103 est remplacé par deux orifices 160 et 161 ménagés dans la partie proximale 102 et par lesquels transite le passe-fil 140. En exerçant une traction sur le passe-fil 140, le crochet 105 passe successivement par l'orifice 160 puis l'orifice 161, ce qui après serrage assure la ligature de la structure anatomique 110.

## Revendications

1. Dispositif pour ligaturer une structure anatomique oblongue, telle qu'un appendice caecal ou un méso-appendice, ou toutes autres structures anatomiques vasculaires ou tissulaires, pouvant être utilisé sous coelioscopie, qui comprend:
d'une part, un élément de ligature (101) se présentant sous la forme d'une bande souple ayant une partie proximale (102) et une partie distale (104), la partie proximale (102) comportant un passant (103), et la partie distale (104) présentant à son extrémité libre un crochet (105) et étant susceptible de coulisser dans ledit passant (103) après son introduction par l'extrémité distale (106) du passant suite à une traction sur le crochet (105), et
d'autre part, un applicateur (120) pour mettre en place cet élément de ligature, ledit applicateur comportant :
- un tube (121) cylindrique ayant une extrémité distale (150) et une extrémité proximale, l'applicateur comportant en outre
- une tige (122) métallique rectiligne qui s'étend dans ledit tube (121) et qui comporte à son extrémité distale (123) une lame ressort (124) qui, en l'absence de contrainte, forme pratiquement un cercle dans le prolongement de ladite tige (122) et dont le diamètre est compris entre cinq et douze fois le diamètre interne dudit tube (121), ladite lame (124) étant susceptible de loger dans ledit tube à l'état - déployé et de reprendre sa forme circulaire lorsqu'on la ressort par l'extrémité distale (150) dudit tube (121),
**caractérisé en ce que** l'applicateur (120) comporte en outre
- des moyens (130) pour retenir, au moins durant son transport, ledit élément de ligature (101) sur la face interne de ladite lame ressort (124), le passant (103) pouvant être disposé à la partie distale (123) de ladite tige (122), et
- un passe-fil (140) pouvant transiter par ledit passant (103) et formant à l'avant de l'extrémité distale (106) dudit passant (103) une ganse (141) dans laquelle peut s'introduire l'extrémité distale (104) de l'élément de ligature (101) lorsque la lame ressort (124) forme un cercle, afin qu'une traction sur ledit passe-fil (140) entraîne le crochetage de ladite ganse (141) par le crochet (105), l'introduction de la partie distale (104) de l'élément de ligature (101) dans ledit passant (103) et le serrage dudit élément de ligature.

2. Dispositif selon la revendication 1, **caractérisé par le fait que** les moyens pour retenir l'élément de ligature (101) comporte une gaine (130) en matériau souple présentant un conduit radialement extérieur (131) dans lequel loge au moins la lame ressort (124) et une rainure (132) radialement intérieure dans laquelle loge au moins en partie l'élément de ligature (101), ladite rainure (132) étant délimitée par deux lèvres (133, 134) retenant les bords longitudinaux dudit élément de ligature (101) et étant susceptibles de s'écarter pour libérer ledit élément de ligature (101) lors de son serrage.

3. Dispositif selon l'une des revendications 1 ou 2, **caractérisé par le fait que** le passant (103) est constitué par deux orifices (160, 161) ménagés dans la partie proximale (102) de l'élément de ligature (101) et par lesquels passe le passe-fil (140).

4. Dispositif selon l'une quelconque des revendications 1 à 3, **caractérisé par le fait que** l'extrémité distale (150) du tube (121) comporte en outre des moyens de découpe pour découper la portion distale (104) de l'élément de ligature (101) après serrage de ce dernier.

5. Dispositif selon la revendication 4, **caractérisé par le fait que** les moyens de découpe sont formés par les bords tranchants (152) d'une encoche (151) prévue à l'extrémité distale (150) du tube (121).

## Patentansprüche

1. Vorrichtung zum Unterbinden einer länglichen anatomischen Struktur, wie eines Wurmfortsatzes oder eines Mesoappendix, oder allen anderen anatomischen Gefäß- oder Gewebestrukturen, die unter Zölioskopie verwendet werden kann, umfassend:
einerseits ein Ligaturelement (101), das in Form eines flexiblen Bandes mit einem proximalen Teil (102) und einem distalen Teil (104) vorliegt, wobei der proximale Teil (102) eine Schlaufe (103) umfaßt und wobei der distale Teil (104) an seinem freien Ende einen Haken (105) aufweist und geeignet ist, nach seinem Einführen über das distale Ende (106) der Schlaufe, infolge eines Ziehens an dem Haken (105), in der Schlaufe (103) zu gleiten, und
andererseits einen Applikator (120), um dieses Ligaturelement anzubringen, wobei der Applikator umfaßt:
- eine zylindrische Röhre (121) mit einem distalen Ende (150) und einem proximalen Ende,
wobei der Applikator ferner umfaßt
- eine geradlinige Metallstange (122), die sich in der Röhre (121) erstreckt und die an ihrem distalen Ende (123) eine Federzunge (124) umfaßt, welche, wenn keine Spannung vorhanden ist, praktisch einen Kreis in der Verlängerung der Stange (122) bildet und deren Durchmesser zwischen dem Fünf- und Zwölffachen des Innendurchmessers der Röhre (121) beträgt, wobei die Zunge (124) geeignet ist, in nicht entfaltetem Zustand in der Röhre untergebracht zu werden und ihre Kreisform wieder anzunehmen, wenn sie über das distale Ende (150) der Röhre (121) ausgefahren wird,
**dadurch gekennzeichnet, daß** der Applikator (120) ferner umfaßt
- Mittel (130), um das Ligaturelement (101) wenigstens während seines Transports an der Innenseite der Federzunge (124) zu halten, wobei die Schlaufe (103) an dem distalen Teil (123) der Stange (122) angeordnet sein kann, und
- einen Fadenführer (140), der die Schlaufe (103) durchqueren kann und der am vorderen Teil des distalen Endes (106) der Schlaufe (103) eine Schlinge (141) bildet, in die das distale Ende (104) des Ligaturelements (101) eindringen kann, wenn die Federzunge (124) einen Kreis bildet, damit ein Ziehen an dem Fadenführer (140) das Anhaken der Schlinge (141) durch den Haken (105), das Einführen des distalen Teils (104) des Ligaturelements (101) in die Schlaufe (103) sowie das Festziehen des Ligaturelements bewirkt.

2. Vorrichtung nach Anspruch 1, **dadurch gekennzeichnet, daß** die Mittel zum Halten des Ligaturelements (101) eine Hülle (130) aus flexiblem Material mit einem radial äußeren Kanal (131), in dem wenigstens die Federzunge (124) untergebracht ist, und einer radial inneren Nut (132), in der wenigstens teilweise das Ligaturelement (101) untergebracht ist, umfassen, wobei die Nut (132) durch zwei Lippen (133, 134) begrenzt ist, die die Längsränder des Ligaturelements (101) halten und die geeignet sind, sich voneinander zu entfernen, um das Ligaturelement (101) bei seinem Festziehen freizugeben.

3. Vorrichtung nach einem der Ansprüche 1 oder 2, **dadurch gekennzeichnet, daß** die Schlaufe (103) durch zwei Öffnungen (160, 161) gebildet ist, die in dem proximalen Teil (102) des Ligaturelements (101) ausgebildet sind und durch die der Fadenführer (140) hindurch läuft.

4. Vorrichtung nach einem der Ansprüche 1 bis 3, **dadurch gekennzeichnet, daß** das distale Ende (150) der Röhre (121) ferner Schneidemittel umfaßt, um den distalen Abschnitt (104) des Ligaturelements (101) nach dessen Festziehen abzuschneiden.

5. Vorrichtung nach Anspruch 4, **dadurch gekennzeichnet, daß** die Schneidemittel durch die Schneidkanten (152) einer Kerbe (151), die an dem distalen Ende (150) der Röhre (121) vorgesehen ist, gebildet sind.

## Claims

1. A device for ligating an oblong anatomical structure such as a vermiform appendix or a mesoappendix, or any other vessel or tissue anatomic structures, the device being suitable for being used under celioscopy, comprising:
firstly a ligature element (101) in the form of a flexible strip having a proximal portion (102) and a distal portion (104), the proximal portion (102) having a keeper (103), and the distal portion (104) having a barb (105) at its free end and being suitable for being slidably received in said keeper (103) after it has been inserted via the distal end (106) of the keeper after traction has been exerted on the barb (105); and
secondly, an applicator (120) for putting said ligature element in place, said applicator comprising:
- a cylindrical tube (121) having a distal end (150) and a proximal end,
the applicator (120) further comprising
- a rectilinear metal rod (122) which extends in said tube (121) and which, at its distal end (123) has a spring blade (124) which, in the absence of stress, forms substantially a circle extending from said rod (122) and whose diameter lies in the range five times the inside diameter of said tube (121) to twelve times said inside diameter, said blade (124) being suitable for being received in said tube in the non-deployed state and for resuming its circular shape when it is brought out via the distal end (150) of said tube (121);
**characterized in that** the applicator (120) further comprises
- means (130) for acting, at least while it is being transported, to retains said ligature element (101) on the inside face of said spring blade (124), the keeper (103) being able to be disposed at the distal portion (123) of said rod (122); and
- a feedthrough (140) able to pass through said keeper (103) and, at the front of distal end (106) of said keeper (103), forming an eye (141) into which the distal end (104) of the ligature element (101) can be inserted when the spring blade (124) forms a circle, so that traction exerted on said feedthrough (140) causes said eye (141) to be hooked by the barb (105), causes the distal portion (104) of the ligature element (101) to be inserted into said keeper (103), and causes said ligature element to be tightened.

2. A device according to claim 1, **characterized by** the fact that the means for retaining the ligature element (101) comprise a sheath (130) made of a flexible material and having a radially outer duct (131) in which at least the spring blade (124) is received, and a radially inner guide groove (132) in which the ligature element (101) is received at least in part, said guide groove (132) being defined by two lips (133, 134) retaining the longitudinal edges of said ligature element (101) and being suitable for moving apart to release said ligature element (101) while it is being tightened.

3. A device according to claim 1 or claim 2, **characterized by** the fact that the keeper (103) is constituted by two orifices (160, 161) provided in the proximal portion (102) of the ligature element (101) and via which the feedthrough (140) passes.

4. A device according to any one of claims 1 to 3, **characterized by** the fact that the distal end (150) of the tube (121) is also provided with cutting means for cutting off the distal end (104) of the ligature element (101) after said ligature element has been tightened.

5. A device according to claim 4, **characterized by** the fact that the cutting means are formed by the sharp edges (152) of a notch (151) provided at the distal end (150) of the tube (121).
